(19) Europäisches Patentamt
European Patent Office
Office européen des brevets

(11) Numéro de publication: **0 360 628 A1**

(12) # DEMANDE DE BREVET EUROPEEN

(21) Numéro de dépôt: **89401882.9**

(22) Date de dépôt: **30.06.89**

(51) Int. Cl.[5]: **C 07 C 43/196**
C 07 C 69/54, C 07 C 67/03,
C 07 C 67/08, C 07 C 41/00,
C 08 F 20/28, C 08 F 120/28,
C 08 F 220/28

(30) Priorité: **22.07.88 FR 8809931**

(43) Date de publication de la demande:
**28.03.90 Bulletin 90/13**

(84) Etats contractants désignés:
**AT BE CH DE ES FR GB GR IT LI LU NL SE**

(71) Demandeur: **NORSOLOR, Société Anonyme dite:**
**18 place des Reflets Tour Aurore**
**F-92080 Paris la Défense 2 (FR)**

(72) Inventeur: **Croisy, Jean François**
**84, rue Principale**
**F-57450 Farschviller (FR)**

**Grosius, Paul**
**11, rue Jeanne d'Arc**
**F-57540 Petite Rosselle (FR)**

(74) Mandataire: **Chaillot, Geneviève**
**Cabinet CHAILLOT 21, avenue Louise de Bettignies**
**F-92700 Colombes (FR)**

The title of the invention has been amended (Guidelines for Examination in the EPO, A-III, 7.3).

(54) **Dérivés du norbornane et du diméthanodécahydronaphtalène utiles pour l'obtention de nouveaux polymères.**

(57) Dérivés (I) du norbornylane et du diméthanodécahydronaphtylane de formule :

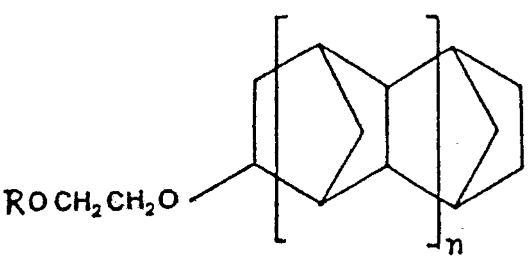

(I)

dans laquelle :
- R est l'atome d'hydrogène quand n est égal à 1,
- ou R est le radical acryloyle ou méthacryloyle quand n est égal à 0 ou 1.

L'invention a également pour objet des procédés de préparation des dérivés (I) et leur application à l'obtention de polymères et copolymères.

EP 0 360 628 A1

## Description

### DERIVES DU NORBORNYLANE ET DU DIMETHANODECAHYDRONAPHTYLANE UTILES POUR L'OBTENTION DE NOUVEAUX POLYMERES.

La présente invention concerne le (diméthanodécahydronaphtyloxy-2)-2 éthanol et les (méth)acrylates de (norbornyloxy-2)-2 éthyle et de (diméthanodécahydronaphtyloxy-2)-2 éthyle, leurs procédés de fabrication et leur application à la synthèse de nouveaux polymères.

La présente invention a pour premier objet de nouveaux dérivés du norbornylane et du diméthanodécahydronaphtylane ayant pour formule :

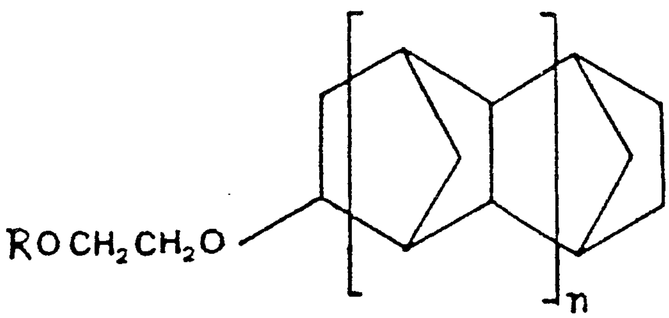

(I)

dans laquelle :

- R est l'atome d'hydrogène quand n est égal à 1, ou bien
- R est le radical acryloyle ou méthacryloyle quand n est égal à 0 ou 1.

A température ambiante, les dérivés (I) se présentent comme des liquides incolores ou jaunes pâles et relativement visqueux. Ils sont miscibles avec de nombreux solvants organiques, comme les hydrocarbures aliphatiques et aromatiques, les alcools et les cétones, par exemple le cyclohexane, le xylène, l'éthanol, la méthylisobutylcétone. Ils forment une émulsion avec l'eau.

Le (diméthanodécahydronaphtyloxy-2)-2 éthanol peut être préparé de différentes façons. De préference, il est préparé par réaction du diméthanodécahydronaphtalène et d'éthylène glycol en présence d'un catalyseur acide, par exemple un acide de Brönstedt tel que l'acide sulfurique ou l'acide paratoluènesulfonique, ou un acide de Lewis tel que les complexes du trifluorure de bore ou du trichlorure d'aluminium ou d'une résine échangeuses d'ions telle que les résines sulfoniques ou perfluorosulfoniques. Cette réaction est effectuée à une température généralement comprise entre 40°C et 130°C.

Les dérivés (I) comportant une fonction (méth)acrylique sont préparés soit par estérification directe soit par transestérification.

La transestérification peut être effectuée à partir d'un (méth)acrylate d'alkyle tel que le méthacrylate de méthyle ou l'acrylate d'éthyle, avec l'alcool correspondant, à savoir selon les cas le (norbornyloxy-2)-2 éthanol ou le (diméthanodécahydronaphtyloxy-2)-2 éthanol respectivement selon que n est égal à 0 ou 1. Elle est généralement effectuée en présence d'au moins un inhibiteur de polymérisation à raison d'au moins 200 ppm et d'au moins un catalyseur de transestérification à raison d'au moins 0,3 % en poids par rapport à l'alcool, à une température généralement comprise entre 60°C et 140°C.

L'estérification directe est réalisée en faisant réagir l'acide (méth)acrylique avec respectivement, selon que n est égal à 0 ou 1, le (norbornyloxy-2)-2 éthanol ou le (diméthanodécahydronaphtyloxy-2)-2 éthanol. Elle est généralement effectuée en présence d'au moins un inhibiteur de polymérisation à raison d'au moins 200 ppm et d'au moins catalyseur d'estérification à raison d'au moins 0,3 % en poids par rapport à l'alcool, à une température généralement comprise entre 40°C et 100°C. De préference, la réaction est effectuée en présence d'au moins un solvant organique, par exemple le cyclohexane, l'heptane ou le toluène, afin d'éliminer l'eau formée par distillation azéotropique.

Parmi les inhibiteurs de polymérisation convenant aux procédés de transestérification et d'estérification précités, on peut citer la phénothiazine, le tertiobutylcatéchol, l'éther méthylique de l'hydroquinone, l'hydroquinone, le bleu de méthylène, les sulfates de cuivre et de fer.

Comme catalyseur de transestérification, on peut utiliser les titanates d'alkyle tels que le titanate d'éthyle ou des complexes du zirconium tels que le tétraacétylacétonate de zirconium. Comme catalyseurs d'estérification directe conviennent des acides comme l'acide paratoluène sulfonique, l'acide méthane sulfonique, ou l'acide sulfurique.

Les dérivés (I) selon l'invention ont diverses applications.

Le (diméthanodécahydronaphtyloxy-2)-2 éthanol est essentiellement un intermédiaire réactionnel utile notamment pour préparer les dérivés (I) comportant une fonction (méth)acrylique. Ceux-ci peuvent se polymériser ou se copolymériser avec d'autres monomères à insaturation éthylénique, tels que l'éthylène, les hydrocarbures vinylaromatiques tels que le styrène, le vinyltoluène ou l'alphaméthylstyrène, et les composés acryliques ou méthacry-liques tels que les (méth)acrylates d'alkyle dont le groupe alkyle possède de 1 à 20 atomes de carbone, les (méth)acrylamides, les (méth)acrylates de dialkylaminoalkyle et leurs sels

quaternaires et de manière générale tous monomères à insaturation éthylénique capables de se copolymériser par un processus radicalaire sous l'effet d'un générateur de radicaux libres tel que micro-ondes, rayonnement bêta, gamma ou ultraviolet ou initiateur chimique tel que persulfate, peroxyde, hydroperoxyde ou composé diazoïque.

Ainsi un second objet de la présente invention consiste en un polymère comprenant dans sa chaîne au moins un motif dérivé d'un (méth)acrylate de (norbornyloxy-2)-2 éthyle ou de (diméthanodécahydronaphtyloxy-2)-2 éthyle et, le cas échéant, au moins un motif dérivé d'un autre monomère à insaturation éthylénique tel que décrit ci-dessus. Dans le cas des copolymères, les conditions de polymérisation seront choisies les plus proches possibles de celles habituellement employées pour la polymérisation du comonomère à insaturation éthylénique, à savoir par exemple :

- une température de 140° C à 300° C et une pression de 1 000 à 3 000 bars environ lorsque ce comonomère est l'éthylène,
- une température de 30° C à 90° C environ lorsque ce comonomère est un composé acrylique ou méthacrylique,
- une température de 80° C à 200° C environ lorsque ce comonomère est un hydrocarbure vinylaromatique.

Les exemples donnés ci-dessous à titre indicatif permettront de mieux comprendre l'invention.

EXEMPLE 1 :

Dans un réacteur à double enveloppe surmonté d'un agitateur mécanique, d'un réfrigérant et équipé d'un dispositif de mesure de la température, on introduit 62,1 g d'éthylène glycol et 10,7 g d'une résine échangeuse d'ions de dénomination commerciale AMBERLYST 15. Dès que la température dans le réacteur atteint 90° C, on ajoute progressivement 160 g de diméthanodécahydronaphtalène en 4 heures.

La réaction est effectuée à une température de 90° C. Après 7 heures de réaction, on filtre le catalyseur et on distille le mélange réactionnel. Le (diméthanodécahydronaphtyloxy-2)-2 éthanol est obtenu à 175° C sous $1,07.10^3$ Pa (8 mm Hg) avec un rendement de 96,1%. Il se présente sous la forme d'un liquide incolore, visqueux avec un indice de réfraction $n_D^{20}$ égal à 1,5218.

Les spectres RMN du carbone 13 et infra-rouge confirment l'obtention d'un mélange des isomères exo (71%) et endo (29%) du (diméthanodécahydronaphtyloxy-2)-2 éthanol.

EXEMPLE 2 :

Dans un appareillage identique à celui décrit dans l'exemple 1 et surmonté d'une colonne de distillation avec tête de reflux permettant une distillation azéotropique, on introduit la charge suivante :

| | |
|---|---|
| - acide méthacrylique (g) | 172,2 |
| - (diméthanodécahydronaphtyloxy-2)-2 éthanol (g) | 110 |
| - éther méthylique de l'hydroquinone (g) | 0,14 |
| - sulfate du cuivre (g) | 0,17 |
| - acide paratoluène sulfonique (g) | 6,9 |
| - cyclohexane (ml) | 200 |

La réaction est effectuée à 80° C sous la pression atmosphérique. Après 4 heures de réaction, on neutralise l'excès d'acide et on élimine le cyclohexane sous vide. On obtient le méthacrylate de (diméthanodécahydronaphtyloxy-2)-2 éthyle avec un rendement de 97%, le produit obtenu ayant une pureté de 93% (mesurée par chromatographie en phase gazeuse).

Le produit obtenu est un liquide jaune pâle.

EXEMPLE 3 :

Dans un appareillage identique à celui décrit dans l'exemple 2, on introduit la charge suivante :

| | |
|---|---|
| - acide acrylique (g) | 114,1 |
| - (diméthanodécahydronaphtyloxy-2)-2 éthanol (g) | 111 |
| - éther méthylique de l'hydroquinone (g) | 0,11 |
| - sulfate du cuivre (g) | 0,14 |
| - acide méthane sulfonique (g) | 5,76 |
| - cyclohexane (ml) | 200 |

La réaction est effectuée à 80° C sous pression atmosphérique. Après 4 heures de réaction, on neutralise l'excès d'acide et on élimine le cyclohexane. On obtient l'acrylate de (diméthanodécahydronaphtyloxy-2)-2 éthyle avec un rendement de 95%, le produit obtenu ayant une pureté de 94% (mesurée par chromatographie en phase gazeuse). Le produit obtenu est un liquide jaune pâle.

EXEMPLE 4 :

a) Préparation du (norbornyloxy-2)-2 éthanol exo

Dans un appareillage identique à celui décrit dans l'exemple 1, on introduit 94 g de norbornène que l'on préchauffe. Une fois le norbornène liquide, on ajoute 93 g d'éthylène glycol et 10,6 g de résine AMBERLYST 15. On obtient alors un mélange biphasique. Après 5 heures de réaction, on filtre la résine et on distille le mélange réactionnel. Le (norbornyloxy-2)-2 éthanol exo est obtenu à 127°C sous 3,33. $10^3$ Pa (25 mm Hg) avec un rendement de 83%. Il se présente sous la forme d'un liquide incolore dont l'indice de réfraction est égal à 1,4788 à 20°C et dont la densité est égale à 1,04 g/$cm^3$ à 23,5°C.

b) Préparation du méthacrylate de (norbornyloxy-2)-2 éthyle exo :

Dans un appareillage identique à celui de l'exemple 2, on introduit la charge suivante :

| | |
|---|---|
| - méthacrylate de méthyle (g) | 200 |
| - (norbornyloxy-2)-2 éthanol exo (g) | 156 |
| - éther méthylique de l'hydroquinone (g) | 0,16 |
| - titanate d'éthyle (g) | 2,74 |

La réaction est effectuée à 90°C. Après 8 heures réaction, on distille le méthacrylate de méthyle en excès puis le méthacrylate de (norbornyloxy-2)-2 éthyle à 135°C sous 0,8.$10^3$ Pa (6 mm Hg).

Le rendement de la réaction est de 84,9%. Le méthacrylate de (norbornyloxy-2)-2 éthyle se présente sous la forme d'un liquide incolore, visqueux avec un indice de réfraction à 20°C de 1,4767.

EXEMPLE 5 :

Dans un appareillage identique à celui de l'exemple 2, On introduit la charge suivante :

| | |
|---|---|
| - acrylate d'éthyle (g) | 200 |
| - (norbornyloxy-2)-2 éthanol exo (g) | 156 |
| - tétraacétylacétonate de zirconium (g) | 1,46 |
| - éther méthylique d'hydroquinone (g) | 0,16 |

La réaction est effectuée à 90°C. Après 8 heures de réaction l'excès d'acrylate d'éthyle est distillé et l'acrylate de (norbornyloxy-2)-2 éthyle exo est obtenu à 125°C sous 0,8. $10^3$ Pa (6 mm Hg) avec un rendement de 98%. Il se présente sous la forme d'un liquide incolore, d'indice de réfraction à 20°C égal à 1,4775.

L,acrylate et le méthacrylate de (norbornyloxy-2)-2 éthyle endo sont obtenus en mélange, respectivement avec l'acrylate et le méthacrylate de (norbornyloxy-2)-2 éthyle exo, par exemple, en utilisant les modes opératoires décrits dans les exemples 4 et 5 ci-dessus et en utilisant comme matière première le (norbornyloxy-2)-2 éthanol endo. Ce dernier est obtenu par réduction de l'éthylène-cétal du norbornylane par le système $LiAlH_4$-$AlCl_3$ (Can. J. Chem. 44/13, 1547-50, (1966)).

**Revendications**

1 - Dérivés (I) du norbornylane et du diméthanodécahydronaphtylane de formule :

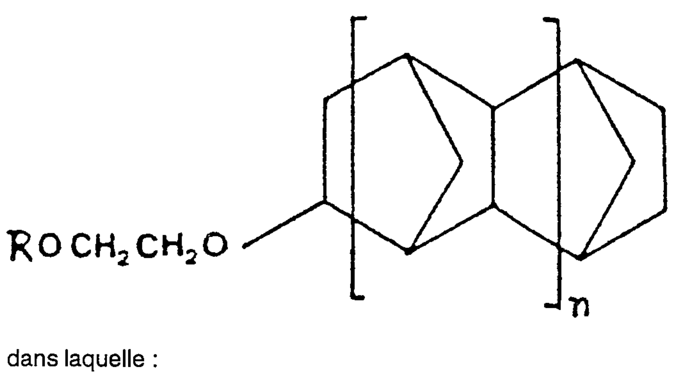

(I)

dans laquelle :

- R est l'atome d'hydrogène quand n est égal à 1,
- ou R est le radical acryloyle ou méthacryloyle quand n est égal à 0 ou 1.

2 - Procédé de préparation du dérivé (I) selon la revendication 1 pour lequel n est égal à 1 et R est l'atome d'hydrogène, par réaction du diméthanodécahydronaphtalène et d'éthylène glycol en présence d'un catalyseur acide.

3 - Procédé selon la revendication 2, caractérisé en ce que ladite réaction est effectuée à une température comprise entre 40° C et 130° C.

4 - Procédé de préparation des derivés (I) selon la revendication 1, pour lesquels R est le radical acryloyle ou méthacryloyle et n est égal à 0 ou 1, selon lequel on fait réagir un méthacrylate d'alkyle avec l'alcool correspondant, à savoir selon le cas le (norbornyloxy-2)-2 éthanol ou le (diméthanodécahydronaphtyloxy-2)-2 éthanol respectivement selon que n est égal à 0 ou 1.

5 - Procédé selon la revendication 4, caractérisé en ce que la réaction est effectuée à une température comprise entre 60° C et 140° C.

6 - Procédé de préparation des dérivés (I) selon la revendication 1, pour lesquels R est le radical acryloyle ou méthacryloyle et n est égal à 0 ou 1, selon lequel on fait réagir l'acide (méth)acrylique avec respectivement, selon que n est égal à 0 ou 1, le (norbornyloxy-2)-2 éthanol ou le (diméthanodécahydronaphtyloxy-2)-2 éthanol, en présence d'un solvant organique.

7 - Procédé selon la revendication 6, caractérisé en ce que la réaction est effectuée à une température comprise entre 40° C et 100° C.

8 - Procédé selon l'une des revendications 4 à 7, caractérisé en ce que la réaction est effectuée en présence d'au moins un inhibiteur de polymérisation.

9 - Procédé selon l'une des revendications 4 à 8, caractérisé en ce que la réaction est effectuée en présence d'au moins un catalyseur.

10 - Polymère comprenant dans sa chaîne au moins un motif dérivé d'un (méth)acrylate de (norbornyloxy-2)-2 éthyle ou de (diméthanodécahydronaphtyloxy-2)-2 éthyle et, le cas échéant, au moins un motif dérivé d'un autre monomère à insaturation éthylénique choisi parmi l'éthylène, les hydrocarbures vinylaromatiques et les composés acryliques ou méthacryliques.

Office européen des brevets

# RAPPORT DE RECHERCHE EUROPEENNE

Numero de la demande

EP 89 40 1882

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-3 412 159 (J. W. H. FABER et al.)<br>* colonne 2; example 3 * | 1-3 | C 07 C 43/196<br>C 07 C 69/54<br>C 07 C 67/03<br>C 07 C 67/08<br>C 07 C 41/00<br>C 08 F 20/28<br>C 08 F 120/28<br>C 08 F 220/28 |
| A | FR-A-2 077 988 (EASTMAN KODAK CO.)<br>* page 1, lignes 1-3; page 6, lignes 11-14; page 11,12, tableau 1, position 5-9 * | 1,4,10 | |
| A | US-A-4 450 307 (P. H. MOSS et al.)<br>* colonne 4, lignes 7-68 * | 1-3 | |
| A | US-A-4 097 677 (W. D. EMMONS et al.)<br>* colonne 1, ligne 48 - colonne 2, ligne 60 * | 1,10 | |
| A | US-A-4 620 028 (C. R. GORMAN et al.)<br>* colonne 3, examples 1,2 * | 1-3 | |
| A | EP-A-0 096 196 (BAYER AG)<br>* page 5, composé XV; pages 16,17, example 3a.b * | 1-5 | **DOMAINES TECHNIQUES RECHERCHES (Int. Cl.4)** |
| A | GB-A-1 012 817 (DISTILLERS CO. LTD.)<br>* revendication 1 * | 4,9 | C 07 C 43/00<br>C 07 C 69/00<br>C 08 F 20/00<br>C 08 F 120/00<br>C 08 F 220/00 |
| A | PATENT ABSTRACTS OF JAPAN<br>vol. 7, no. 219 (C-188)[1364] 29 septembre 1983; & JP - A - 58 117207 (MATSUSHITA DENKO K.K.) 12.07.1983 | 1,10 | |
| A | PATENT ABSTRACTS OF JAPAN<br>vol. 9, no. 287 (C-314)[2010] 14 novembre 1985; & JP - A 60 130608 (HITACHI KASAI KOGYO K.K.) 12.07.85) | 1,10 | |

Le présent rapport a été établi pour toutes les revendications

| Lieu de la recherche | Date d'achèvement de la recherche | Examinateur |
|---|---|---|
| BERLIN | 28-08-1989 | PROBERT C.L. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)